# EUROPÄISCHE PATENTSCHRIFT

(11) **EP 0 950 751 B1**
(45) Veröffentlichungstag und Bekanntmachung des Hinweises auf die Patenterteilung: **13.10.2004**
(21) Anmeldenummer: 99810285.9
(22) Anmeldetag: 07.04.1999
(51) Int. Cl.: D06M 13/358, C07D 251/44, C07D 251/16

(54) **Verfahren zur Behandlung von Cellulosefasern**
Process for the treatment of cellulose fibers
Procédé de traitement de fibres de cellulose

(30) Priorität: 14.04.1998 EP 98810316
(43) Veröffentlichungstag der Anmeldung: 20.10.1999
(73) Patentinhaber: LENZING AKTIENGESELLSCHAFT, 4860 Lenzing (AT)
(72) Erfinder: Aeschlimann, Peter, 4123 Allschwil (CH)
(74) Vertreter: Schwarz, Albin, Dr.

(56) Entgegenhaltungen:
- EP-A- 0 036 133
- EP-A- 0 538 977
- EP-A- 0 882 836
- EP-A- 0 903 434
- WO-A-91/10006
- WO-A-94/09191
- DE-A- 19 611 668
- FR-A- 1 243 816

## Beschreibung

Die vorliegende Erfindung betrifft ein Verfahren zur Verringerung der Fibrillierungsneigung bei Lyocell-Cellulosefasern.

Als Lyocell werden solche Faser bezeichnet, die durch ein Verfahren erhalten werden, bei dem die Cellulose in einem organischen Lösungsmittel, einer Kombination eines organischen Lösungsmittels mit einem anorganischen Salz oder in wässrigen Salzlösungen gelöst, und anschliessend aus dieser Lösung gesponnen wird.

Die Brauchbarkeit von aus den genannten Fasern hergestellten Flächengebilden, z.B. Textilmaterialien, wird jedoch durch die ausgeprägte Neigung dieser Fasern, im nassen Zustand zu fibrillieren, stark eingeschränkt. Unter Fibrillierung wird das Aufbrechen der nassen Faser in Längsrichtung bei z.B. mechanischer Beanspruchung bis zum Ablösen von Fibrillen entlang der Faseroberfläche verstanden, wodurch die Faser ein haariges oder pelziges Aussehens erhält. Ein aus diesen Fasern hergestelltes Gewebe verliert ausserdem nach einigen Wäschen stark an Farbintensität.

Es besteht somit ein Bedarf nach einem Verfahren, das die Fibrillierung bei Lyocell-Fasern verringert oder diese vollständig unterdrückt.

Es hat sich überaschenderweise gezeigt, dass durch das erfindungsgemässe Verfahren die Fibrillierungsneigung der behandelten Lyocell-Fasern stark verringert wird.

Gegenstand der vorliegenden Erfindung ist somit ein Verfahren zur Behandlung von Lyocell-Cellulosefasern, dadurch gekennzeichnet, dass man die Lyocell-Cellulosefaser mit mindestens einer Verbindung der Formel 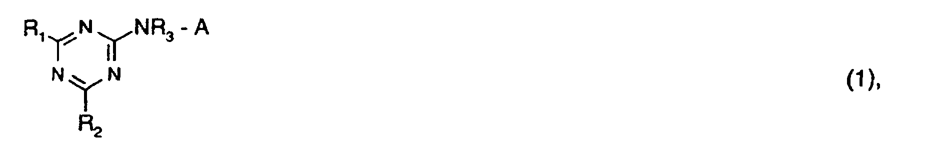 behandelt, worin
A ein unsubstituiertes oder durch Hydroxy, Carboxy, -NH-CO-CBr=CH₂ oder
-NH-CO-CHBr-CH₂Br substituiertes Phenyl, oder ein unsubstituiertes oder durch Hydroxy, Carboxy, Sulfo, -SO₂-CH=CH₂, -SO₂-CH₂CH₂-OSO₃H, -NH-CO-CBr=CH₂ oder -NH-CO-CHBr-CH₂Br substituiertes Naphthyl ist,
R₁ Halogen ist,
R₂ Halogen oder ein unsubstituiertes oder durch Hydroxy, Carboxy, -NH-CO-CBr=CH₂ oder -NH-CO-CHBr-CH₂Br substituiertes Phenyl, oder ein unsubstituiertes oder durch Hydroxy, Carboxy, Sulfo, -SO₂-CH=CH₂, -SO₂-CH₂CH₂-OSO₃H, -NH-CO-CBr=CH₂ oder -NH-CO-CHBr-CH₂Br substituiertes Naphthyl ist, und
R₃ Wasserstoff oder unsubstituiertes oder durch Hydroxy oder Sulfo substituiertes C₁-C₄-Alkyl ist,
mit der Massgabe, dass entweder R₁ und R₂ Halogen bedeuten und A und R₃ die angegebenen Bedeutungen haben, oder A und R₂ Phenyl oder Naphthyl bedeuten und mindestens zwei voneinander unabhängige Substituenten, ausgewählt aus der Gruppe, enthaltend -SO₂ CH=CH₂, -SO₂-CH₂CH₂-OSO₃H, -NH-CO-CBr=CH₂ und -NH-CO-CHBr-CH₂Br, enthalten müssen.

R₃ als C₁-C₄-Alkyl ist Methyl, Ethyl, Propyl, iso-Propyl, n-Butyl, sec.-Butyl, iso-Butyl oder tert.-Butyl.

Halogen als Rest R₁ und R₂ bedeutet unabhängig voneinander Fluor, oder insbesondere Chlor.

Für das erfindungsgemässe Verfahren sind von besonderem Interesse die Verbindungen der Formel (1), worin R₂ Halogen ist.

Von ganz besonderem Interesse für das erfindungsgemässe Verfahren sind die Verbindungen der Formel (1), worin R₁ und R₂ Chlor sind.

Wichtig für das erfindungsgemässe Verfahren sind die Verbindungen der Formel (1), worin R₁ und R₂ Chlor sind, -N-R₃ -NH-, -N(CH₂CH₂OH)-, -N(CH₂SO₃H)- oder -N(CH₂CH₃)- ist, und A ein Rest der Formel 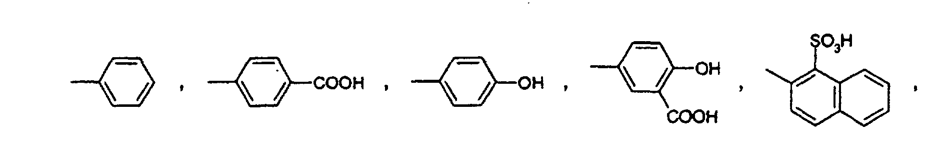 oder 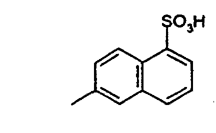 ist.

Besonders wichtig für das erfindungsgemässe Verfahren sind die Verbindungen der Formel 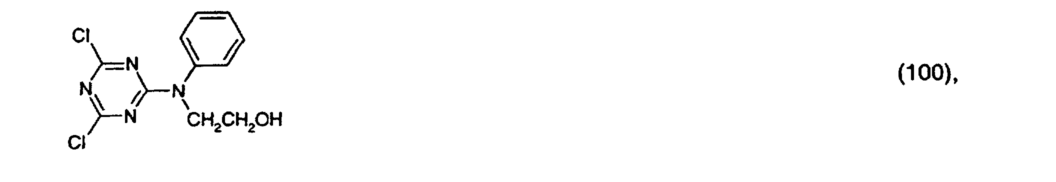 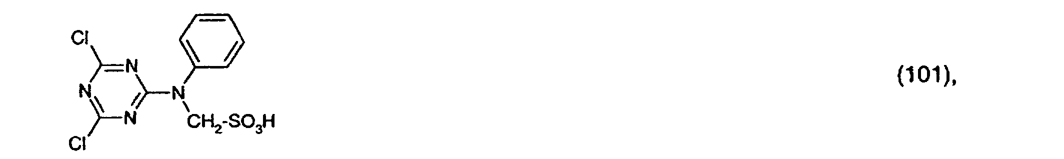 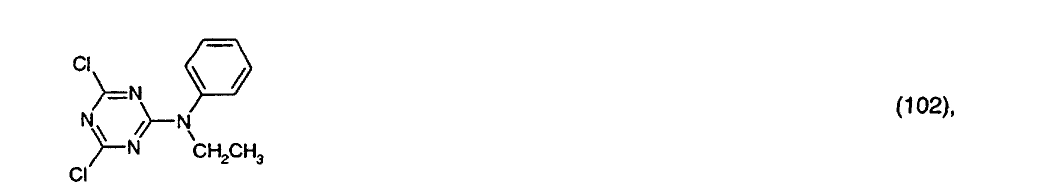 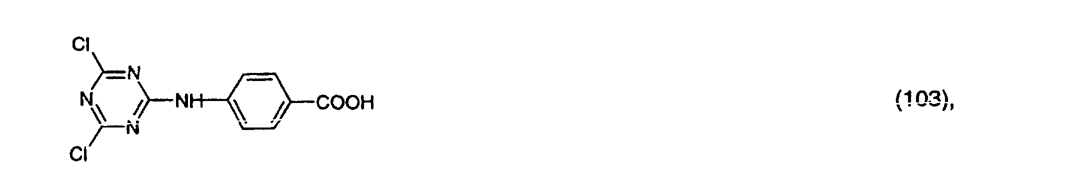 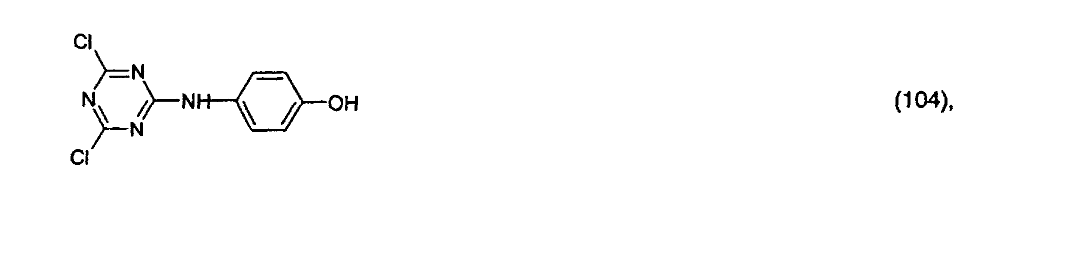 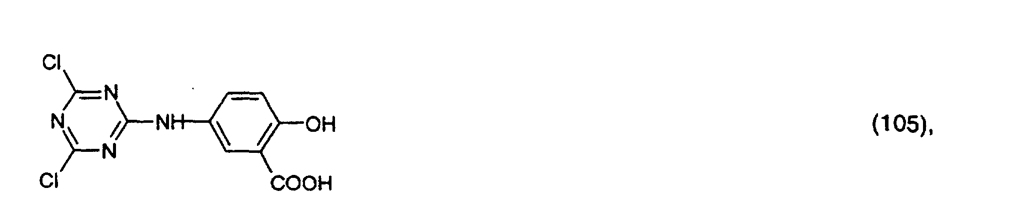 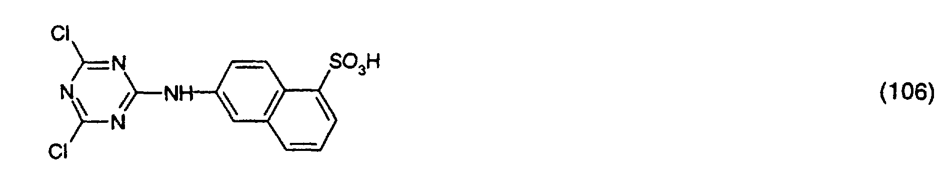 und

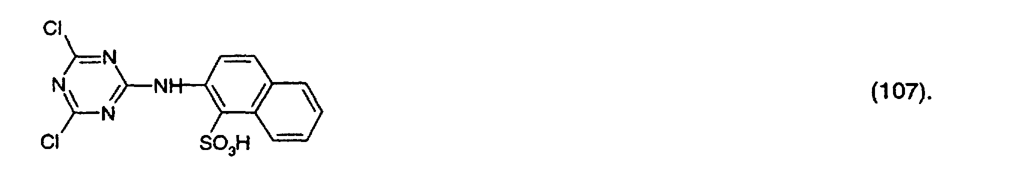

Ganz besonders wichtig für das erfindungsgemässe Verfahren sind die Verbindungen der Formeln (103), (106) und (107).

Die Verbindungen der Formeln (106) und (107) sind neu und stellen einen weiteren Gegenstand der vorliegenden Erfindung dar.

Die Herstellung der in dem erfindungsgemässen Verfahren verwendeten Verbindungen der Formel (1), geschieht nach an sich bekannten Methoden, indem man z.B. ein Trichlortriazin der Formel 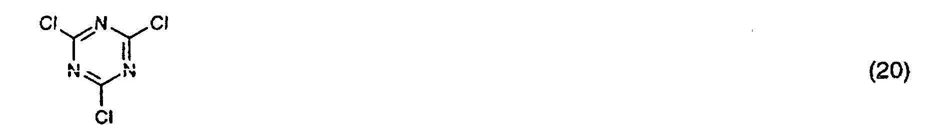 mit äquimolarer Menge einer Verbindung der Formel

NHR₃-A (21)

worin R₃ und A die unter der Formel (1) angegebene Bedeutung haben, umsetzt und das entstehende Endprodukt der Formel (1) isoliert.

Die Herstellung der neuen Verbindungen der Formeln (106) und (107) geschieht in analoger Weise, indem man ein Trichlortriazin der Formel (20) mit einem Amin der Formel 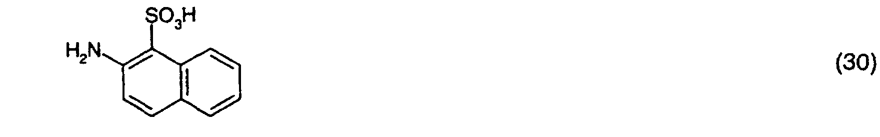 oder 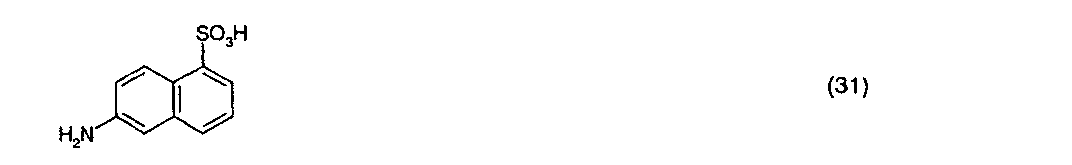 umsetzt.

Die Amine der Formeln (30) und (31) sind bekannt und können nach an sich bekannten Methoden hergestellt werden.

Eine zweckmässige Ausführungsform des erfindungsgemässen Verfahrens ist dadurch gekennzeichnet, dass die Lyocell-Cellulosefasern mit den erfindungsgemäss verwendeten Verbindungen in einem alkalischen Milieu behandelt werden.
Das alkalische Milieu wird vorzugsweise von einem Alkylicarbonat und/oder einem Alkalihydroxid, wie z.B. Natriumcarbonat, Natriumhydroxid oder Kaliumhydroxid, gebildet.

Die Behandlung kann dabei sowohl an ungefärbten Fasern, als auch an zu färbenden Fasern vor, während oder unmittelbar nach einem Färbeprozess, durchgeführt werden.

Werden die erfindungsgemäss verwendeten Verbindungen an ungefärbten Fasern appliziert, so kann es entweder mittels eines Behandlungsbades oder direkt nach dem Spinnvorgang an den frisch gesponnenen, noch nicht getrockneten Fasern (sog. "never dried"-Faser, wie sie z.B. in der EP-A-0 538 977 auf der Seite 4, oder in der US-A-5,580,354 in der Spalte 4 beschrieben sind) geschehen.

Werden die erfindungsgemäss verwendeten Verbindungen im Laufe eines Färbeprozesses eingesetzt, so geschieht dies vorteilhafterweise vor oder während des eigentlichen Färbevorganges.

Die erfindungsgemäss verwendeten Verbindungen können dabei in einem separaten Behandlungsbad; oder vorzugsweise zusammen mit den verwendeten Farbstoffen in einem Färbebad bei den jeweiligen Färbebedingungen auf die Faser appliziert werden.
Werden die erfindungsgemäss verwendeten Verbindungen aus einem separaten Behandlungsbad auf die Faser gebracht, so kann dies bei einer Temperatur von 15 bis 140° C, vorzugsweise von 40 bis 100° C während 10 bis 120 Minuten, vorzugsweise 30 bis 90 Minuten, erfolgen. Eine weitere vorteilhafte Ausführung des erfindungsgemässen Verfahrens besteht darin, die zu behandelnden Faser mit einer wässrigen Lösung der erfindungsgemäss verwendeten Verbindungen zu benetzen und anschliessend während 5 bis 60, vorzugsweise 10 bis 30 Sekunden einer Dampfbehandlung bei 90 bis 130° C zu unterziehen.
Die Benetzung der Faser kann sowohl durch Eintauchen in ein Bad als auch durch Besprühen der Faser stattfinden.

Die Lyocell-Cellulosefasern können als solche, als Garn, oder in Form von Flächengebilden, wie z.B. Gewebe, Gestricke oder Gewirke vorliegen und behandelt werden.

Die Mengen in denen die erfindungsgemäss verwendeten Verbindungen in den Behandlungs- oder Färbeflotten eingesetzt werden, liegen normalerweise zwischen 0,1 und 15 Gew.-%, insbesondere zwischen 1 und 10 Gew.-%, vor allem zwischen 2 und 6 Gew.-% bezogen auf das Gewicht der Faser.

Einen weiteren Gegenstand der vorliegenden Erfindung stellt die Verwendung der Verbindungen der Formel (1) zur Verringerung der Fibrillierung bei der Lyocell-Cellulose dar.
Die Fibrillierungneigung der unbehandelten und behandelten Fasern wird nach einem modifiziertem Martindale Scheuertest, bei dem das genässte Gewebemuster bis zur ersten Lochbildung unter definierter Belastung gescheuert wird, beurteilt. Die Anzahl der Scheuertouren bis zur Lochbildung ergibt die Nassscheuerbeständigkeit, die als Massstab für die Fibrillierung dient.

Die nachfolgende Beispiele dienen der Erläuterung der Erfindung. Die Temperaturen sind in Celsiusgraden angegeben, Teile sind Gewichtsteile, und Prozentangaben beziehen sich auf Gew.-%, sofern nicht anders vermerkt. Gewichtsteile stehen zu Volumenteilen im Verhältnis von Kilogramm zu Liter.

### Beispiel 1:

18,5 g Cyanurchlorid werden in einem Laborreaktionsgefäss in einer Mischung von 100 g Eis und 100 g Wasser, welche 0,1 g eines handelsüblichen oberflächenwirksamen Hilfsmittels enthält, intensiv verrührt.
Anschliessend wird eine neutrale Lösung von 13,7 g 4-Aminobenzoesäure zugegesetzt und die Temperatur bei 0°C und der pH-Wert bei 6,0 gehalten.
Nachdem mittels einer HPLC-Probe kein Amin nachgewiesen werden kann, wird das Reaktionsprodukt durch Aussalzen ausgefällt und durch Filtration isoliert. Nach anschliessender Trocknung bei 30°C erhält man die Verbindung der Formel

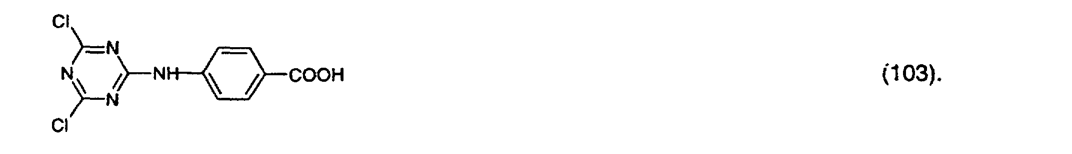

### Beispiele 2 bis 8:

Verfährt man wie im Beispiel 1 angegeben, verwendet aber anstatt 13,7 g 4-Aminobenzoesäure die äquimolare Menge einer der Verbindungen der Formeln (50) bis (55), (30) und (31) so erhält man die in der Tabelle 1 aufgeführten Verbindungen der Formeln (100) bis (102) bzw. (104) bis (107).

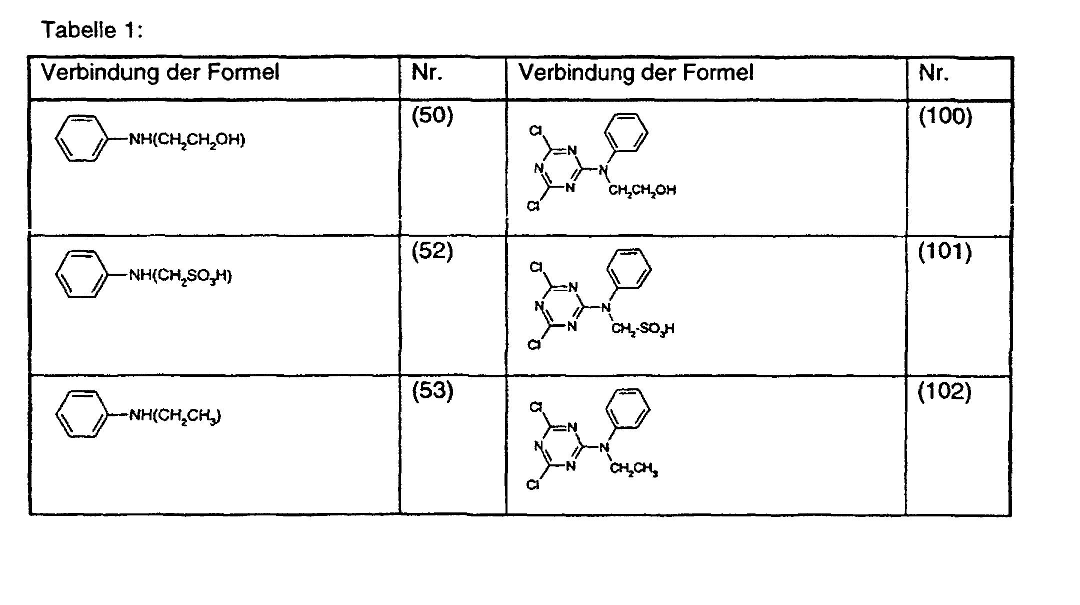

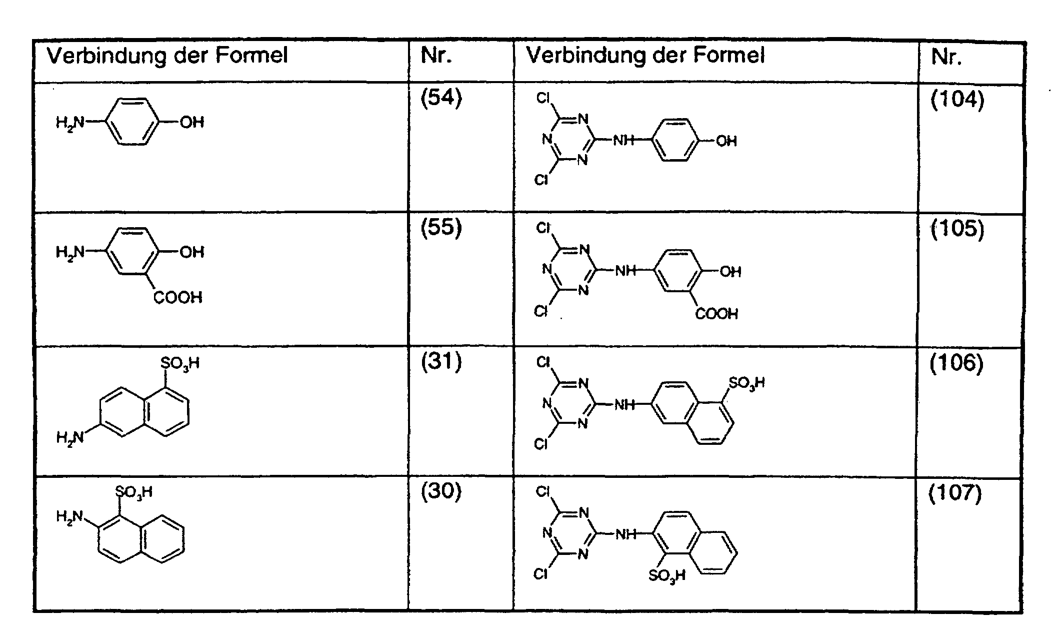

### Beispiel 9:

In einer Laborfärbeapparatur wird eine Flotte, bestehend aus
50 ml Wasser und
15 ml einer 20%-igen wässrigen Lösung von Natriumsulfat,
vorgelegt und auf 50°C aufgeheizt. Danach wird in die Flotte ein Stück von 10 g Lyocell-Gewebe eingetaucht und die Temperatur mit einem Gradient von 2°C/Min auf 90°C erhöht.
Während der Aufheizphase werden bei 70°C 0,6 g der Verbindung der Formel (107), gelöst in 27,5 ml Wasser, zugegeben. Das Lyocell-Gewebe wird bei 90°C 60 Minuten behandelt.
Anschliessend wird die Behandlungsflotte auf 70°C abgekühlt, versetzt mit 7,5 ml einer 20%igen wässrigen Soda-Lösung und weitere 45 Minuten bei 70°C gehalten.
Danach wird das Bad abgelassen und das behandelte Lyocell-Gewebe mit Wasser gespült, in frischem, nur aus Wasser bestehendem, Bad 5 Minuten gekocht, nochmals kalt gespült und getrocknet.
Das getrocknete Lyocell-Gewebe zeigt bei einem Martindale-Scheuertest eine um ca. 60% grössere Anzahl Scheuertouren im Vergleich zu unbehandeltem Lyocell-Gewebe.

Bei dieser Applikation kann das Lyocell-Gewebe auch gleichzeitig gefärbt werden, indem ein oder mehrere Reaktivfarbstoffe direkt nach Zugabe der Verbindung der Formel (107) zugegeben werden.

Verfährt man wie im Beispiel 9 beschrieben, verwendet aber anstatt 0,6 g der Verbindung der Formel (107) die gleiche Menge einer der Verbindungen der Formeln (101), (103), (104), (105) und (106), so erhält man ebenfalls ein Lyocell-Gewebe mit hohen Martindale-Scheuertestwerten.

### Beispiel 10:

In einer Laborfärbeapparatur wird eine Flotte, bestehend aus
50 ml Wasser und
15 ml einer 20%-igen wässrigen Lösung von Natriumsulfat,
vorgelegt und auf 60°C aufgeheizt. Danach wird in die Flotte ein Stück von 10 g Lyocell-Gewebe eingetaucht und 3 Minuten später 0,6 g der Verbindung der Formel (106), gelöst in 25,5 ml Wasser zugegeben. Nach weiteren 15 Minuten wird die Flotte mit 7,5 ml einer 20% igen wässrigen Soda-Lösung versetzt. Danach hält man die Flotte 30 Minuten bei 60°C.
Dann versetzt man die Flotte mit 2 ml einer 3%-igen wässrigen NaOH-Lösung, hält weitere 10 Minuten bei 60°C und stellt anschliessend das Lyocell-Gewebe wie in Beispiel 9 beschrieben fertig.
Das fertiggestellte Lyocell-Gewebe kann anschliessend nach üblichen Verfahren, mit z.B. Reaktivfarbstoffen, gefärbt werden.

Das getrocknete Lyocell-Gewebe zeigt bei einem Martindale-Scheuertest eine um ca. 60% grössere Anzahl Scheuertouren im Vergleich zu unbehandeltem Lyocell-Gewebe.

Verfährt man wie im Beispiel 10 beschrieben, verwendet aber anstatt 0,6 g der Verbindung der Formel (106) die gleiche Menge einer der Verbindungen der Formeln (101), (103), (104), (105) und (107), so erhält man ebenfalls ein Lyocell-Gewebe mit hohen Martindale-Scheuertestwerten.

### Beispiel 11:

Eine Flotte, enthaltend
42 g/l einer Verbindung der Formel 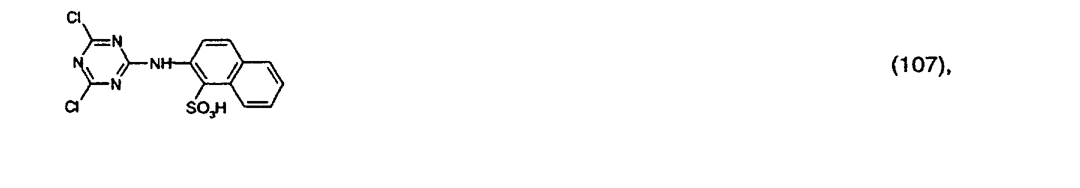 30 g/l kalziniertes Natriumcarbonat und
1 g/l eines handelsüblichen Netzmittels,
wird auf ein Lyocell-Gewebe auffoulardiert (Flottenaufnahme 70%). Das so behandelte Lyocell-Gewebe wird ohne Trocknung direkt während 8 Minuten in Sattdampf von 102° C weiterbehandelt. Danach wird das Lyocell-Gewebe mit Wasser gespült, in frischem, rein wässrigen Bad 5 Minuten gekocht, kalt gespült und getrocknet.
Das behandelte Lyocell-Gewebe zeigt bei einem Martindale-Scheuertest im Vergleich zu unbehandeltem Lyocell-Gewebe eine ca.1,5-fache Anzahl Scheuertouren.

## Patentansprüche

1. Verfahren zur Behandlung von Lyocell-Cellulosefasern, **dadurch gekennzeichnet, dass** man die Lyocell-Cellulosefaser mit mindestens einer Verbindung der Formel 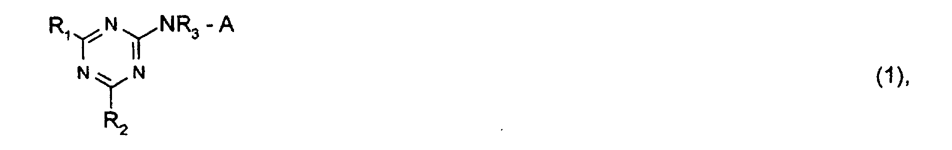 behandelt, worin
A ein unsubstituiertes oder durch Hydroxy, Carboxy, -NH-CO-CBr=CH₂ oder -NH-CO-CHBr-CH₂Br substituiertes Phenyl, oder ein unsubstituiertes oder durch Hydroxy, Carboxy, Sulfo, -SO₂-CH=CH₂, -SO₂-CH₂CH₂-OSO₃H, -NH-CO-CBr=CH₂ oder -NH-CO-CHBr-CH₂Br substituiertes Naphthyl ist, R₁ Halogen ist, R₂ Halogen oder ein unsubstituiertes oder durch Hydroxy, Carboxy, -NH-CO-CBr=CH₂ oder -NH-CO-CHBr-CH₂Br substituiertes Phenyl, oder ein unsubstituiertes oder durch Hydroxy, Carboxy, Sulfo, -SO₂-CH=CH₂, -SO₂-CH₂CH₂-OSO₃H, -NH-CO-CBr=CH₂ oder -NH-CO-CHBr-CH₂Br substituiertes Naphthyl ist, und R₃ Wasserstoff oder unsubstituiertes oder durch Hydroxy oder Sulfo substituiertes C₁-C₄-Alkyl ist,
mit der Massgabe, dass entweder R₁ und R₂ Halogen bedeuten und A und R₃ die angegebenen Bedeutungen haben, oder A und R₂ Phenyl oder Naphthyl bedeuten und mindestens zwei voneinander unabhängige Substituenten, ausgewählt aus der Gruppe, enthaltend -SO₂ CH=CH₂, -SO₂-CH₂CH₂-OSO₃H, -NH-CO-CBr=CH₂ und -NH-CO-CHBr-CH₂Br, enthalten müssen.

2. Verfahren gemäss Anspruch 1, **dadurch gekennzeichnet, dass** man die Lyocell-Cellulosefaser mit mindestens einer Verbindung der Formel (1) behandelt, worin R₁ und R₂ Chlor sind, -N-R₃
-NH-, -N(CH₂CH₂OH)-, -N(CH₂SO₃H)- oder -N(CH₂CH₃)- ist, und A 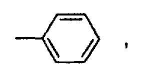 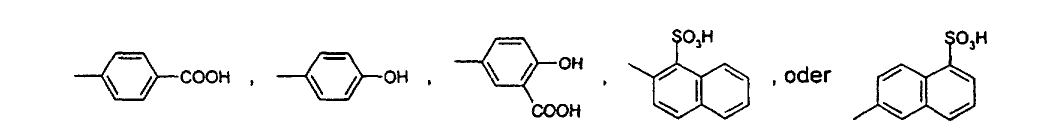 ist.

3. Verfahren gemäss Anspruch 2, **dadurch gekennzeichnet, dass** man die Lyocell-Cellulosefaser mit mindestens einer Verbindung der Formel 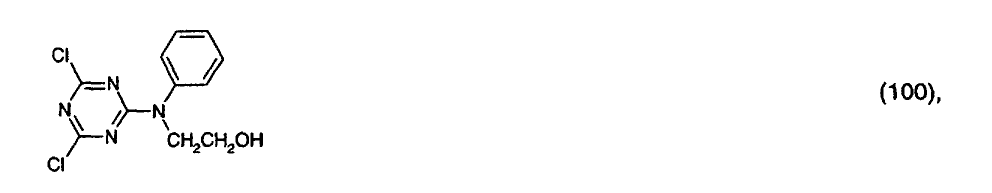 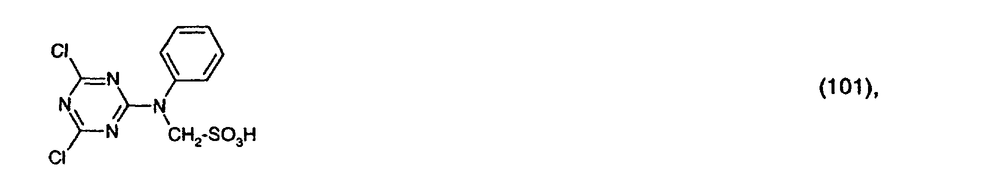 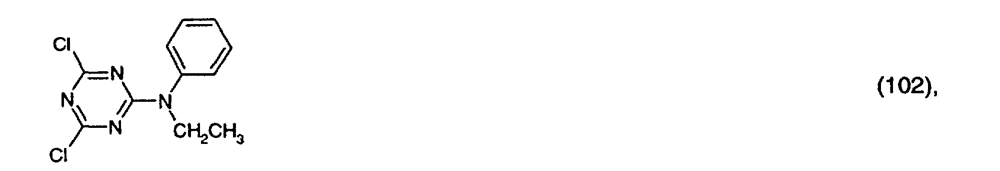 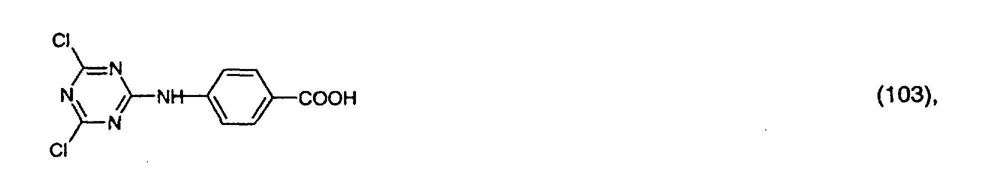 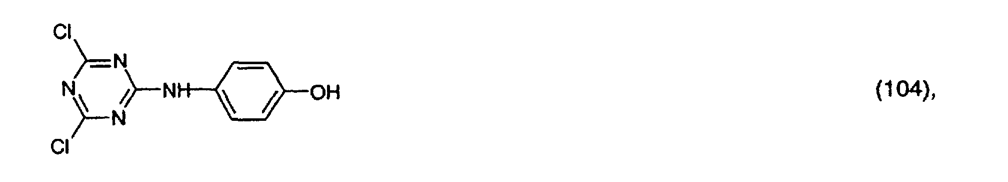 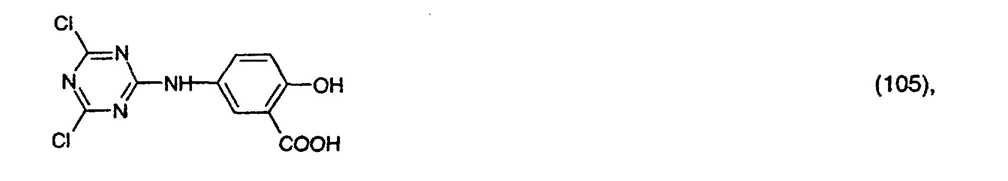 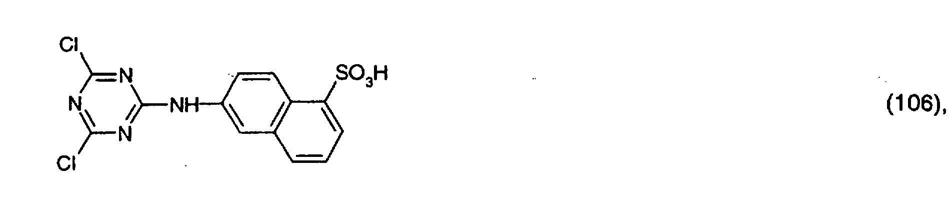 oder 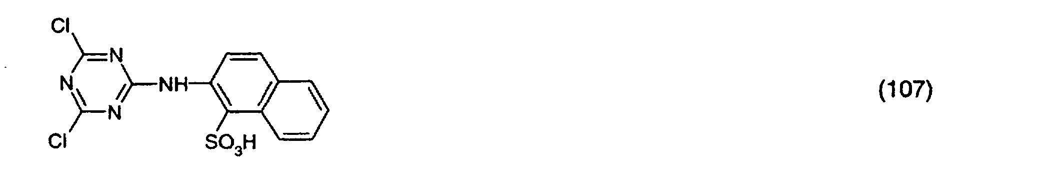 behandelt.

4. Verfahren gemäss Anspruch 1, **dadurch gekennzeichnet, dass** man zwischen 0,1 und 15 Gew.-% der Verbindung der Formel (1), bezogen auf das Gewicht der Faser, verwendet.

5. Verfahren gemäss Anspruch 3, **dadurch gekennzeichnet, dass** man zwischen 0,1 und 15 Gew.-% einer der Verbindungen der Formel (100) bis (108), bezogen auf das Gewicht der Faser, verwendet.

6. Verfahren gemäss Anspruch 1, **dadurch gekennzeichnet, dass** man die Verbindung der Formel (1) an die frisch gesponnenen, noch nicht getrockneten Faser, appliziert.

7. Verfahren gemäss Anspruch 1, **dadurch gekennzeichnet, dass** man die Verbindung der Formel (1) vor oder während eines Färbeprozesses auf die Faser appliziert.

8. Verbindungen gemäss Anspruch 1 der Formel 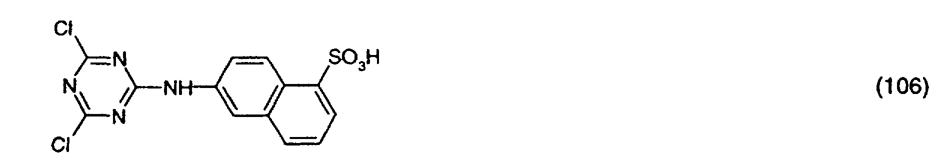 und

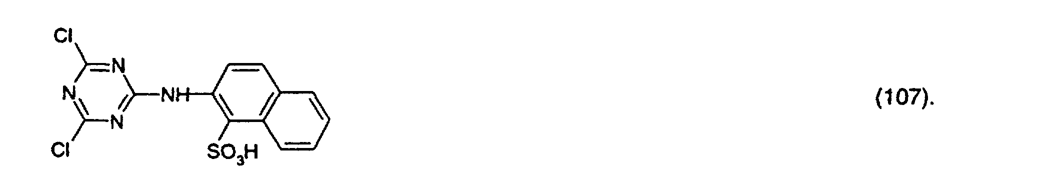

9. Verfahren zur Herstellung der Verbindungen der Formeln (106) und (107) gemäss Anspruch 8, **dadurch gekennzeichnet, dass** man ein Trichlortriazin der Formel 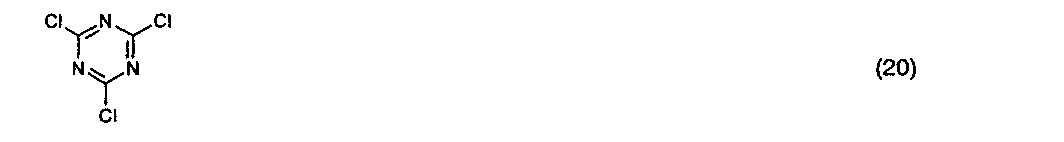 mit einer Verbindung der Formel 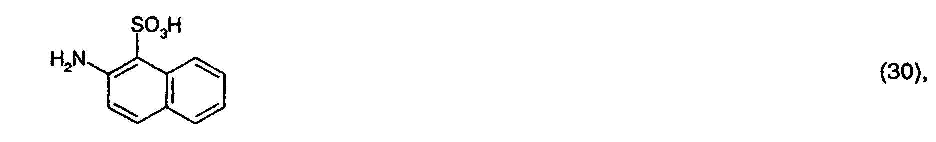 oder 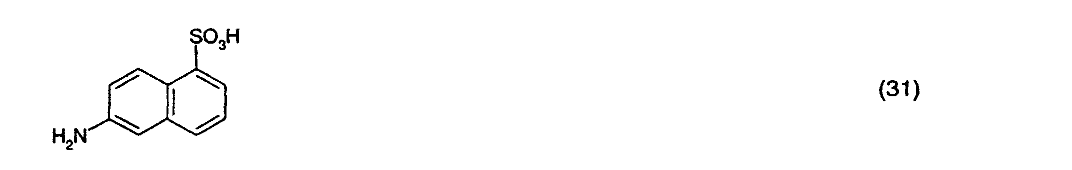 umsetzt.

10. Verwendung der Vebindungen gemäss Anspruch 1 zur Verringerung der Fibrillierung bei Lyocell-Cellulosefasern.

## Claims

1. A process for the treatment of Lyocell cellulose fibres, **characterized in that** the Lyocell cellulose fibre is treated with at least one compound of the formula 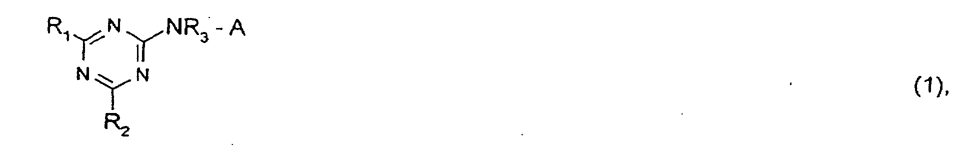 wherein
A is a phenyl that is unsubstituted or substituted by hydroxy, carboxy, -NH-CO-CBr=CH₂ or -NH-CO-CHBr-CH₂Br, or a naphthyl that is unsubstituted or substituted by hydroxy, carboxy, sulfo, -SO₂-CH=CH₂, -SO₂-CH₂CH₂-OSO₃H, -NH-CO-CBr=CH₂ or -NH-CO-CHBr-CH₂Br, R₁ is halogen, R₂ is halogen or a phenyl that is unsubstituted or substituted by hydroxy, carboxy, -NH-CO-CBr=CH₂ or -NH-CO-CHBr-CH₂Br, or a naphthyl that is unsubstituted or substituted by hydroxy, carboxy, sulfo, -SO₂-CH=CH₂, -SO₂-CH₂CH₂-OSO₃H, -NH-CO-CBr=CH₂ or -NH-CO-CHBr-CH₂Br, and R₃ is hydrogen or C₁-C₄-alkyl that is unsubstituted or substituted by hydroxy or sulfo,
with the proviso that either R₁ and R₂ denote halogen and A and R₃ have the above-indicated meanings, or that A and R₂ denote phenyl or naphthyl and must contain at least two substituents that are independent of each other and are selected from the group containing -SO₂-CH=CH₂, -SO₂-CH₂CH₂-OSO₃H, -NH-CO-CBr=CH₂ and -NH-CO-CHBr-CH₂Br.

2. A process according to claim 1, **characterized in that** the Lyocell cellulose fibre is treated with at least one compound of formula (1), wherein R₁ and R₂ are chlorine,
-N-R₃ is
- NH-, -N(CH₂CH₂OH)-, -N(CH₂SO₃H)- or -N(CH₂CH₃)-, and A is

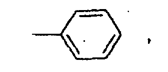

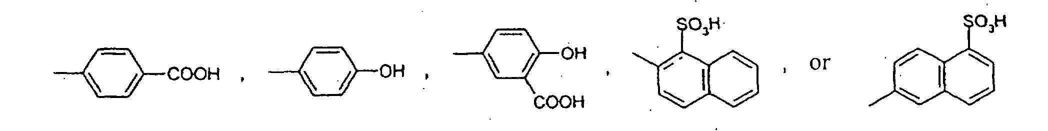

3. A process according to claim 2, **characterized in that** the Lyocell cellulose fibre is treated with at least one compound of the formula 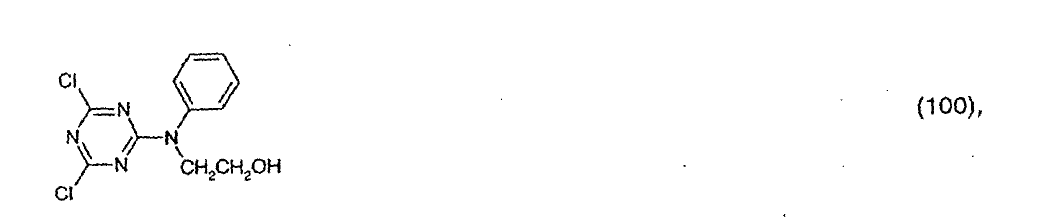 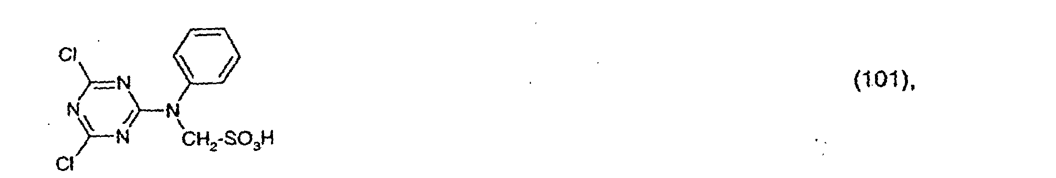 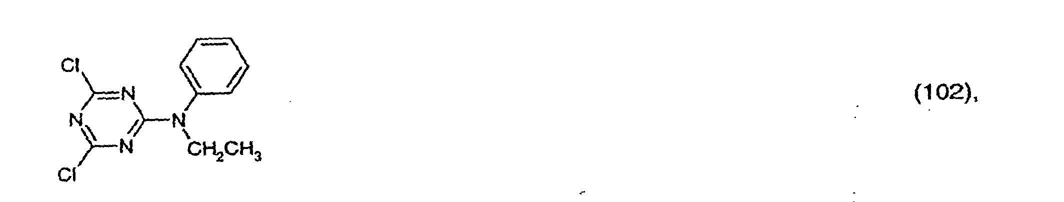 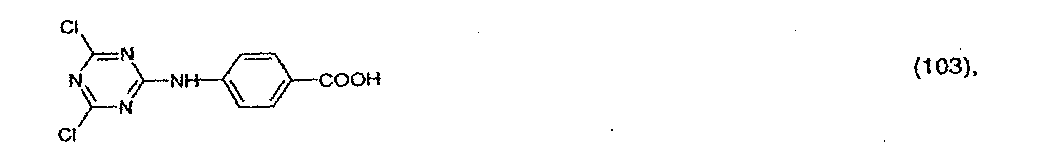 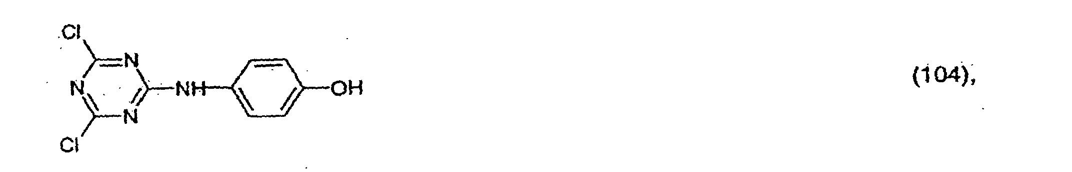 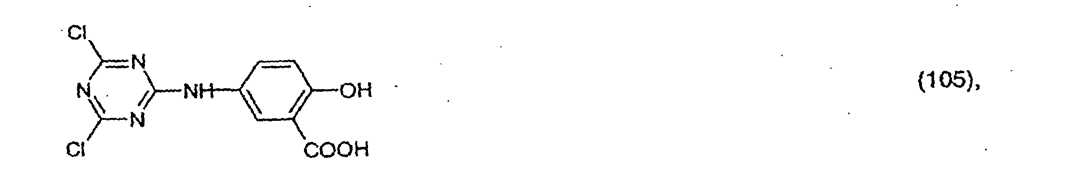 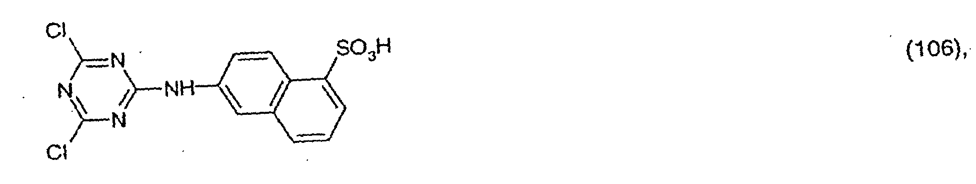 or

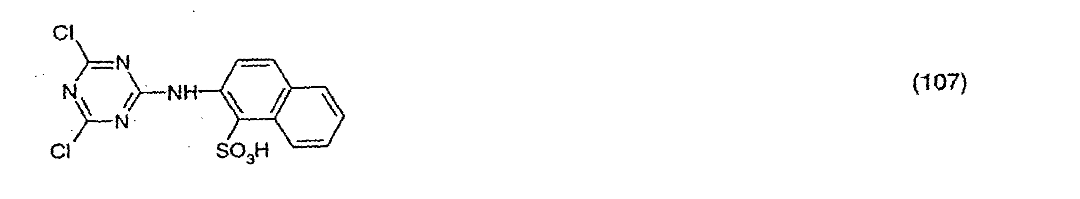

4. A process according to claim 1, **characterized in that** between 0.1 and 15% by weight of the compound of formula (1), based on the weight of the fiber, is used.

5. A process according to claim 3, **characterized in that** between 0.1 and 15% by weight of one of the compounds of formulae (100) to (108), based on the weight of the fiber, is used.

6. A process according to claim 1, **characterized in that** the compound of formula (1) is applied onto the freshly spun, not yet dried fibre.

7. A process according to claim 1, **characterized in that** the compound of formula (1) is applied onto the fibre prior to or during a dyeing process.

8. Compounds according to claim 1 of the formulae 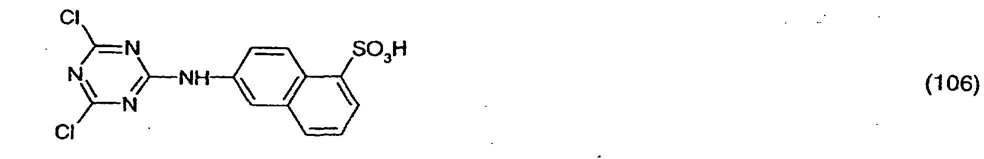 and

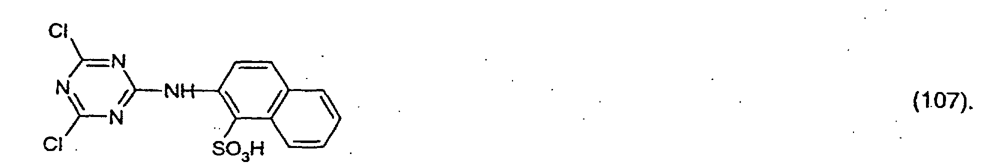

9. A process for preparing the compounds of formulae (106) and (107) according to claim 8, **characterized in that** a trichlorotriazine of the formula 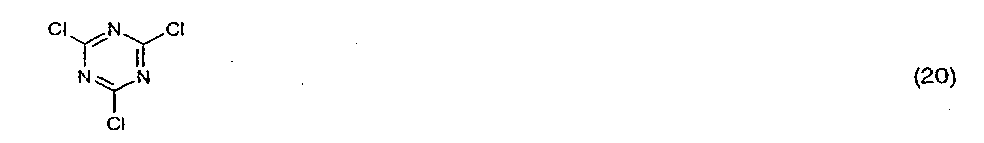 is reacted with a compound of the formula 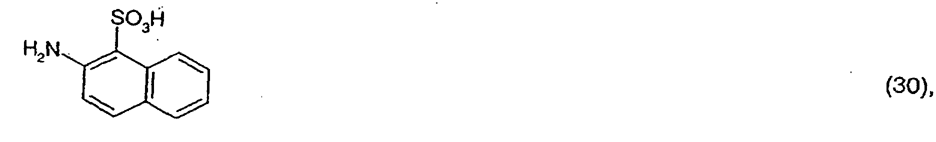 or

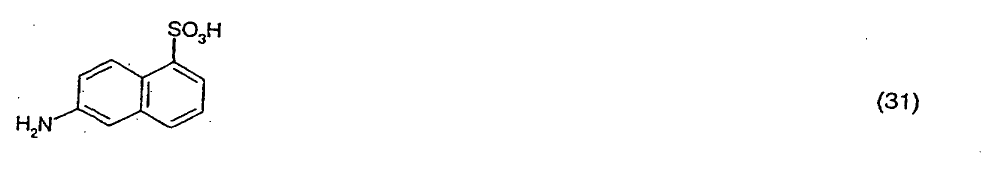

10. The use of the compounds according to claim 1 for decreasing the fibrillation of Lyocell cellulose fibres.

## Revendications

1. Procédé de traitement de fibres de cellulose Lyocell, **caractérisé en ce que** l'on traite les fibres de cellulose Lyocell avec au moins un composé de la formule : 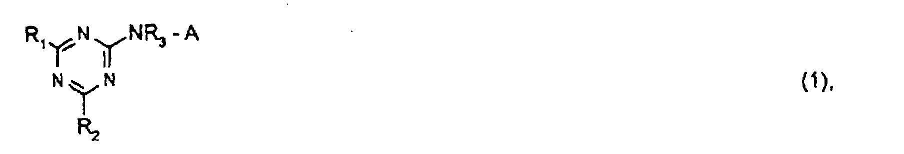 où
A est un phényle non substitué ou substitué par hydroxy, carboxy, -NH-CO-CBr=CH₂ ou -NH-CO-CHBr-CH₂Br, ou un naphtyle non substitué ou substitué par hydroxy, carboxy, sulfo, -SO₂-CH=CH₂, -SO₂-CH₂CH₂-OSO₃H, -NH-CO-CBr=CH₂ ou -NH-CO-CHBr-CH₂Br, R₁ est halogène, R₂ est halogène ou un phényle non substitué ou substitué par hydroxy, carboxy, -NH-CO-CBr=CH₂ ou -NH-CO-CHBr-CH₂Br, ou un naphtyle non substitué ou substitué par hydroxy, carboxy, sulfo, -SO₂-CH=CH₂, -SO₂-CH₂CH₂-OSO₃H, -NH-CO-CBr=CH₂ ou -NH-CO-CHBr-CH₂Br, et R₃ est halogène ou un alkyle en C₁-C₄ non substitué ou substitué par hydroxy ou sulfo,
avec la condition que R₁ et R₂ représentent halogène et A et R₃ ont les mêmes significations, ou que A et R₂ représentent phényle ou naphtyle et doivent contenir au moins deux substituants indépendants l'un de l'autre, choisis parmi le groupe -SO₂-CH=CH₂, -SO₂-CH₂CH₂-OSO₃H, -NH-CO-CBr=CH₂ et -NH-CO-CHBr-CH₂Br.

2. Procédé selon la revendication 1, **caractérisé en ce que** l'on traite les fibres de cellulose Lyocell avec au moins un composé de la formule (1), où R₁ et R₂ sont chlore , -N-R₃ est -NH-, -N(CH₂CH₂OH)-, -N(CH₂SO₃H)- ou -N(CH₂CH₃)-, et A est

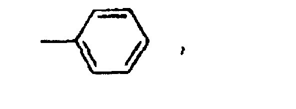

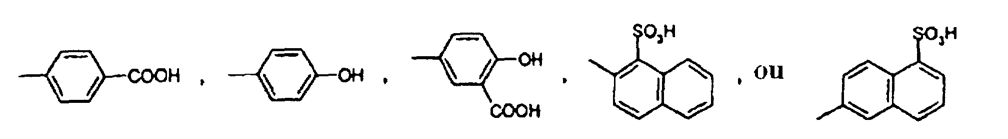

3. Procédé selon la revendication 2, **caractérisé en ce que** l'on traite les fibres de cellulose Lyocell avec au moins un composé de la formule 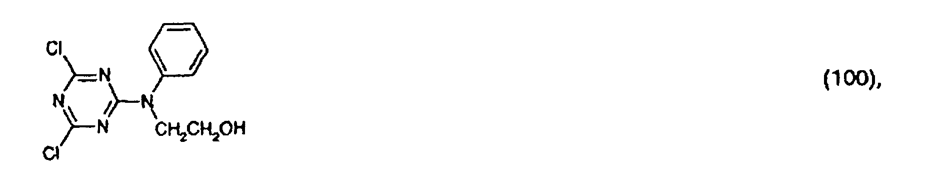 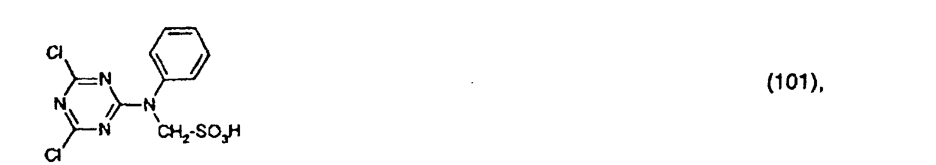 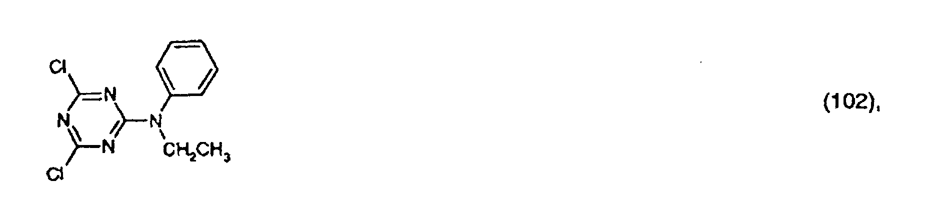 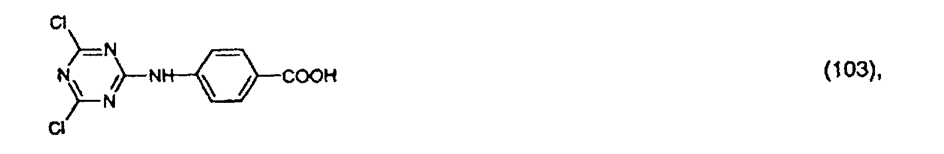 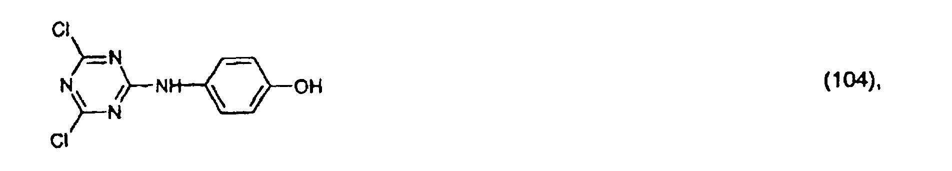 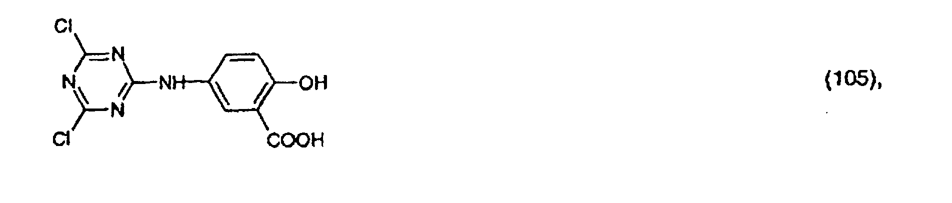 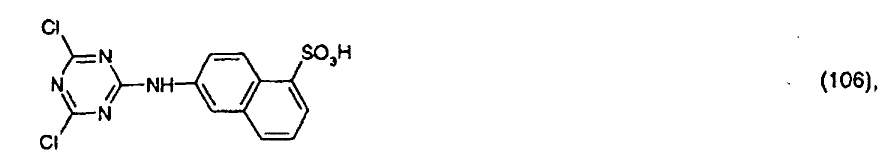 ou

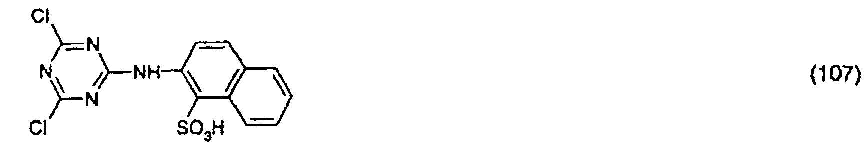

4. Procédé selon la revendication 1, **caractérisé en ce que** l'on utilise de 0,1 à 15% en poids du composé de la formule (1), sur base du poids des fibres.

5. Procédé selon la revendication 3, **caractérisé en ce que** l'on utilise de 0,1 à 15% en poids d'un des composés des formules (100) à (108), sur base du poids des fibres.

6. Procédé selon la revendication 1, **caractérisé en ce que** l'on applique le composé de la formule (1) sur les fibres fraîchement filées, non encore séchées.

7. Procédé selon la revendication 1, **caractérisé en ce que** l'on applique le composé de la formule (1) sur les fibres avant ou pendant un processus de coloration.

8. Composés selon la revendication 1, des formules : 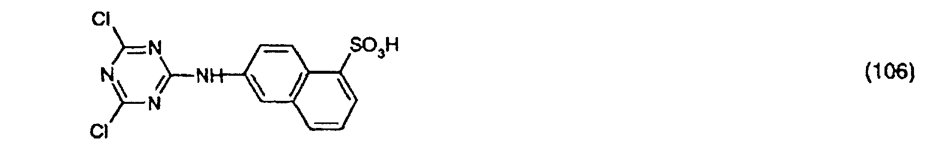 et

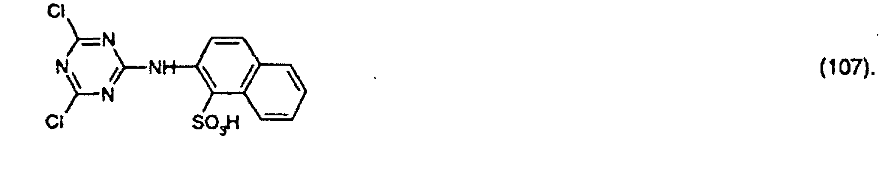

9. Procédé de préparation des composés des formules (106) et (107) selon la revendication 8, **caractérisé en ce que** l'on fait réagir une trichlorotriazine de la formule : 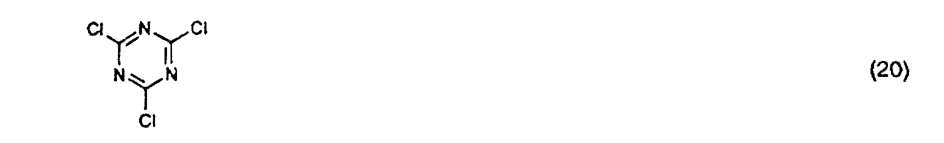 avec un composé de la formule : 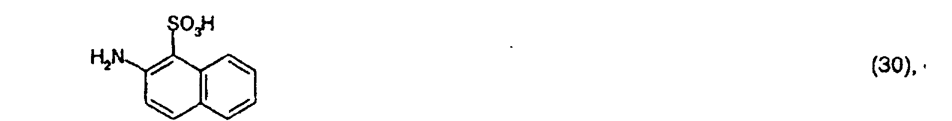 ou

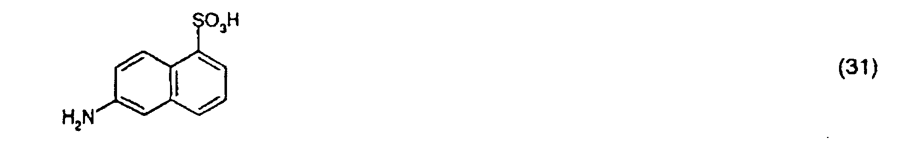

10. Utilisation des composés selon la revendication 1, pour diminuer la fibrillation de fibres de cellulose Lyocell.
